# EUROPEAN PATENT SPECIFICATION

(11) **EP 3 277 384 B1**
(45) Date of publication and mention of the grant of the patent: **13.10.2021**
(21) Application number: 16711315.8
(22) Date of filing: 23.03.2016
(51) Int. Cl.: A61Q 11/00, A61K 8/27, A61K 8/02

(54) **SOLID ORAL CARE COMPOSITIONS**
FESTE MUNDPFLEGEZUSAMMENSETZUNGEN
COMPOSITIONS SOLIDES POUR L'HYGIÈNE BUCCO-DENTAIRE

(30) Priority: 30.03.2015 EP 15161612
(43) Date of publication of application: 07.02.2018
(73) Proprietor: Unilever IP Holdings B.V., 3013 AL Rotterdam (NL); Unilever Global IP Limited, Wirral, CH62 AZD (GB)
(72) Inventor: ADAMS, Suzanne, Elizabeth, Bebington Wirral Merseyside CH63 3JW (GB); ASHCROFT, Alexander, Thomas, Bebington Wirral Merseyside CH63 3JW (GB); EVANS, Rebecca, Mary, Bebington Wirral Merseyside CH63 3JW (GB); GROVES, Brian, Joseph, Bebington Wirral Merseyside CH63 3JW (GB); MCALINDEN, Joanne, Bebington Wirral Merseyside CH63 3JW (GB); RUPARELL, Avika, Melton Mowbray Leicestershire LE13 0NT (GB); WILSON, William, John, Bebington Wirral Merseyside CH63 3JW (GB)
(74) Representative: Tansley, Sally Elizabeth
(86) International application number: PCT/EP2016/056435
(87) International publication number: WO 2016/156161

(56) References cited:
- WO-A2-2007/076001
- GB-A- 1 290 627
- GB-A- 2 187 642
- JP-A- H11 246 375
- EBY ET AL: "Zinc lozenges as cure for the common cold A review and hypothesis", MEDICAL HYPOTHESES, EDEN PRESS, PENRITH, US, vol. 74, no. 3, 1 March 2010 (2010-03-01), pages 482-492, XP026892024, ISSN: 0306-9877, DOI: 10.1016/J.MEHY.2009.10.017 [retrieved on 2009-11-10]

## Description

### Field of the Invention

The present invention is concerned with oral care compositions for cleaning the teeth.

### Background of the Invention

Oral care tablets are forms of dentifrice formulations that use less water when cleaning the teeth and so conserve water.

US 3,431,339 describes a compressed dental tablet for use in place of toothpaste. The tablet contains a blend of a water-soluble anticaries agent, polishing agent, foaming agent and releasable matrix, all in a substantially anhydrous form. Advantages of the format are stated to be improved storage stability of the anticaries agent in the dry tablet and improved cleaning of the lingual surfaces, compared to conventional toothpaste.

US 3,116,208 discloses a dental cleanser in tablet form comprised of calcium carbonate and sodium lauryl sulphate. The tablet may be crushed by the teeth and the sodium lauryl sulphate causes foaming upon brushing the teeth.

US2007/0154409 describes a tooth cleansing tablet consisting of a quantity of tooth powder formed into a firm tablet. The tablet is made from a combination of a mild tooth abrasive with a binding agent and flavouring. The tablet is protected by a coating which provides water resistance and can be made of any ingredient commonly used to form the coatings on candies or gum balls.

US2004/0101494 discloses chewable compressed oral care tablets comprising water soluble gums. The tablets are said to deliver oral care actives directly into the tooth surface.

It is an object of the present invention to provide oral care compositions, in particular tablets with good antimicrobial activity yet which have high flavour intensity, a pleasant taste and leave a fresh feeling in the mouth after use.

### Summary of the Invention

The present invention an oral care composition comprising at least three zinc salts and or zinc oxides:
i) the first at 20°C having a solubility great than 500g/litre in water and is zinc sulphate 7H20;
ii) the second at 20°C having a solubility from 0.05g/litre to 500g/litre in water and is zinc citrate;
iii) the third at 20°C having a solubility less than 0.05g/litre in water and is zinc oxide.

The invention further relates to said composition for use in a method of cleaning teeth comprising the step applying to the teeth and mouth by chewing a composition as described above.

### Detailed Description of the Invention

The present invention relates to solid oral care compositions, preferably in anhydrous form, more preferably in the form of a tablet, most preferably in the form a compressed tablet.

The composition of the invention is preferably non-aqueous. By "non-aqueous" it is generally meant that water is not deliberately added to the composition in any significant quantity. However, the term "non-aqueous" does not mean that small amounts of water cannot be present, for example as a consequence of its association with hygroscopic raw materials. Accordingly, for the purposes of this invention, the term "non-aqueous" generally means that water is present in an amount no greater than about 5%, more preferably no greater than about 3% by weight based on the total weight of the composition.

In the context of the present invention statements relating to levels of ingredients relate to the broad definitions within the specification and more specific combinations of ingredients with the broad definitions.

Composition according to the invention comprise a combination of zinc salts having differing solubilities.

The first zinc salt has a solubility at 20°C in water of greater than 500g/litre, preferably greater than 1000 g/litre and zinc sulphate 7H₂O.

The second zinc salt has a solubility at 20°C is from 0.01g/litre to 300g/litre in water and is zinc citrate trihydrate.

The solubility of the third zinc salt/oxide at 20°C is preferably less than 0.005g/litre.

The third zinc is zinc oxide.

Preferably the weight ratio of the first zinc salt to the second zinc salt is from 2:1 to 6:1.

Preferably the weight ratio of the first zinc salt to the third zinc salt/oxide is from 6:1 to15:1.

It is preferable if the total level of zinc salt/oxide in the composition is from 0.05 to 12 wt% of the total compositions, more preferably from 0.5 to 8 wt % most preferably form 1 to 5 wt% subject to a preferred maximum inclusion level of 1% of total zinc ions.

Compositions: the composition comprises 0.1 to 5%, by weight, of the first metal salt; and from 0.05 to 2%, by weight, of the second metal salt.

Preferred levels of zinc salts are from 0.5 to 4 wt% zinc sulphate heptahydrate, from 0.1 to 2 wt% zinc oxide and from 0.2 to 2 wt % zinc citrate trihydrate. More preferred levels are from 0.5 to 3 wt% zinc sulphate heptahydrate, from 0.1 to1.2 wt% zinc oxide and from 0.2 to 1.4 wt% zinc citrate trihydrate. Most preferred is from 2 to 3 wt% zinc sulphate from 0.4 to 0.8 wt% zinc citrate trihydrate and from 0.15to 0.4 wt% zinc oxide.

Composition of the invention preferably comprise calcium carbonate, the calcium carbonate being a particulate material which acts to mechanically remove debris from a tooth surface and debride plaque.

Suitable particulate calcium carbonates may be characterised by the specific shape and size of their constituent primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates.

For the purposes of the present invention, a suitable source of particulate calcium carbonate includes crystalline calcium carbonates.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline calcium carbonates suitable for use in the invention are available naturally (through the extraction and processing of natural ores) or synthetically (through chemical precipitation). There are three main crystalline polymorphs: calcite, aragonite, and less commonly found, vaterite. The vaterite form of calcium carbonate is metastable and irreversibly transforms into calcite and aragonite. There are many different morphologies(crystal habits) for each of these crystalline forms. The calcite crystalline polymorph is the most commonly used crystal form of calcium carbonate. Over 300 crystalline forms of calcite have been reported in the literature.

Particulate calcium carbonates suitable for use in the invention (such as the materials described above) have an average particle size which can vary over a wide range. Usually the average particle size is 50 microns or less.

Particle (e.g. crystal) size may be determined by standard techniques known to those skilled in the art such as sedimentation. For the present invention the particle size should be determined using a Malvern mastersizer

Good cleaning performance has been obtained with crystalline calcium carbonates in which at least 50% by weight, preferably at least 80% by weight of the crystals are rhombohedral calcite crystals with an average particle size d(50) of 30 microns or less, preferably 15 microns or less. Most preferable are crystalline calcium carbonates with an average particle size d(50)from 0.1 to 30 microns, preferably 0.1 to 15 microns, most preferably 2 to 10 microns (measured using a Malvern mastersizer).

Suitable sources of crystalline calcium carbonate of the size and shape defined above include natural ground calcium carbonate abrasives, generally obtainable from mining and mechanical grinding of sedimentary rocks such as limestone or chalk, or metamorphic rocks such as marble. Natural calcium carbonate is usually of the calcitic polymorph, with a crystal morphology which may typically be characterised as rhombohedral.

Preferred natural ground calcium carbonate abrasives of the type defined above are selected from ground limestone, chalk or marble, optionally refined or partially refined to remove impurities, and having an average particle size d(50)("median ESD" as described above) of about 30 microns or less, more preferably ranging from about 1 to 15 microns, and most preferably ranging from about 2 to 10 microns. Commercially available examples include those materials sold by the company OMYA™ AG under the names OMYACARB™ 2-AV and OMYACARB™ 5-AV. These are fine ground high purity white marble abrasives having an average particle size ("median ESD" as described above) of about 2 and about 5 microns respectively.

Other types of particulate calcium carbonate may also be suitable, depending on the particular balance of cleaning and abrasion required from a consumer perspective. Mixtures of any of the above described abrasive cleaning agents may also be used.

The total amount of calcium carbonate in the composition of the invention will depend on the particular agent (or agents) used, but generally ranges from 3 to 80%, preferably from 15% to 75%, more preferably from 30% to 70% most preferably from 40 to 50 wt% by weight by total weight of the composition.

Compositions of the invention preferably comprise a carboxy methyl cellulose, preferably sodium carboxy methyl cellulose.

The weight of carboxy methyl cellulose in the composition is preferably from 0.1 to 3 wt%, more preferably from 0.5 to 2 wt% of the total composition.

It is preferable if the polyethylene glycol is present as part of a mixture within the tablet rather than an external coating.

Preferably the weight ratio of carboxy methyl cellulose to calcium carbonate is from 1:20 to 1:100, more preferably from 1:30 to 1:50.

Suitable foaming agents for use in forming the composition include surfactants. Surfactants help to increase the rate of dissolution of the solid oral care composition when in contact with saliva, and assist in foaming of the composition during usage.

Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines.

Preferred surfactants for use in the invention include those anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

Mixtures of any of the above described materials may also be used.

The total amount of surfactant incorporated into the composition of the invention generally ranges from about 0.2 to about 5%, by total weight surfactant based on the total weight of the composition.

The composition may also include various additional ingredients to improve aspects such as ease of processing, product performance and/or consumer acceptability.

For example, the composition may include effervescent agents which promote foaming by the provision of bubbles. Suitable effervescent agents for use in this context include acid/carbonate salt couples which provide effervescence by the reaction of the carbonate salt with the acid. Sodium bicarbonate and sodium carbonate represent preferred carbonate salts. However potassium, ammonium, magnesium, calcium carbonate or other metal or organic salts can also be used. Suitable acids include citric acid, fumaric acid, tartaric acid, malic acid, adipic acid and other edible acids.

The amount of effervescent agent incorporated into the composition of the composition of the invention generally ranges from about 0.5 to about 10%, preferably from about 1 to 7%, by total weight effervescent agent (e.g. acid/carbonate salt couple) based on the total weight of the composition.

Mixtures of any of the above described materials may also be used.

The composition will preferably include one or more organic polyols having 2 or more hydroxyl groups in the molecule (hereinafter termed "organic polyols"). These materials may serve as binders to aid in compression of the composition during processing. They may also act as humectants. Humectants help to keep the composition from hardening or crystallizing upon exposure to air. They also help to give the composition a moist feel to the mouth, and may in some cases impart a desirable sweetness. Suitable organic polyols for use in this context include sucrose, dextrose, maltose, fructose, glycerol, sorbitol, xylitol, mannitol, lactitol, maltitol, isomalt, erythritol, polyethylene glycol, polypropylene glycol, propylene glycol, and hydrogenated partially hydrolyzed polysaccharides such as hydrogenated starch hydrolysates. Preferred organic polyols for use in this context are non-cariogenic polyhydric alcohols such as glycerol, sorbitol, maltitol and xylitol. Xylitol is particularly preferred.

Mixtures of any of the above described materials may also be used.

The amount of organic polyol incorporated into the composition of the composition of the invention generally ranges from about 5 to about 70%, preferably from about 10 to 40%, by total weight organic polyol based on the total weight of the composition.

The solid oral care composition of the invention will generally take the form of a discrete, single unit dose such as a pellet, pastille or tablet. Such a single unit dose will typically range in size from 200 mg to 5000 mg, preferably from 250 mg to 2000 mg, more preferably from 500 to 1500 mg.

Tablets of the invention are preferably manufactured using a compaction process to 1 to 8 tons. Preferably the tablets are not coated.

Compositions according to the invention are particularly suitable as a vehicle for oral care actives which may be physically or chemically incompatible with water, or which may function less efficiently in an aqueous environment.

Specific examples of oral care actives which may be particularly suitable for inclusion in the compositions of the invention are:
oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8-hydroxyquinoline derivatives), lactoperoxidase, lactoferrin, lysozyme and mixtures thereof;
fluoride sources such as sodium fluoride, stannous fluoride, sodium monofluorophosphate, zinc ammonium fluoride, tin ammonium fluoride, calcium fluoride, cobalt ammonium fluoride and mixtures thereof;
plant-derivable antioxidants such as flavonoid, catechin, polyphenol, and tannin compounds and mixtures thereof;
antioxidant vitamins such as tocopherols and/or derivatives thereof, ascorbic acid and/or derivatives thereof and mixtures thereof.
agents for the remineralisation of teeth (i.e the *in situ* generation of hydroxyapatite on teeth), such as a mixture of a calcium source and a phosphate source which, when delivered to the teeth results in the *in situ* generation of hydroxyapatite on teeth.

Mixtures of any of the above described materials may also be used.

Compositions of the present invention may also contain further optional ingredients customary in the art such as anticalculus agents, buffers, flavouring agents, sweetening agents, colouring agents, opacifying agents, preservatives, antisensitivity agents, antimicrobial agents and the like.

The solid oral care composition of the invention is used to clean the surfaces of the oral cavity. The composition is an oral composition that is not intentionally swallowed for purposes of systemic administration of therapeutic agents, but is applied to the oral cavity, used to treat the oral cavity and then expectorated. Typically the composition is used in conjunction with a cleaning implement such as a toothbrush, usually by applying it to the bristles of the toothbrush and then brushing the accessible surfaces of the oral cavity.

The invention is further illustrated with reference to the following, non-limiting Examples. Examples of the invention are illustrated by a number, comparative Examples are illustrated by a letter.

### Examples

Tablets were prepared by preparing a dry mix of ingredients and compressing into a tablet.

**Table 1**

| **Material/Product** | **Ex A** | **Ex 1** | **Ex B** |
|---|---|---|---|
| Sodium saccharin | 0.3 | 0.3 | 0.3 |
| Menthol | 0.5 | 0.5 | 0.5 |
| Empicol LZD | 3 | 3 | 3 |
| Polyox WSR-301 | 0.5 | 0.5 | 0.5 |
| Carrageenan | 0.5 | 0.5 | 0.5 |
| Firmenich standard flavour | 6 | 6 | 6 |
| Xylitol 300 | 21.7 | 21.7 | 21.7 |
| Zinc sulphate heptahydrate | 4.4 | 2.64 | 2.64 |
| Zinc citrate trihydrate | 0 | 0.64 | 1.28 |
| Zinc oxide | 0 | 0.249 | 0 |
| Zinc ascorbate | 0 | 0 | 0 |
| Chalk | 63.1 | 63.971 | 63.58 |
| Total | 100 | 100 | 100 |

**Table 2**

| **Salt** | **Approximate Solubility (g/l at 25C)** |
|---|---|
| Zinc Sulphate.7H₂O | 1667 |
| Zinc Citrate.3H₂O | 0.5-100 |
| Zinc Oxide | 0.005 |

The tablets were assessed by a trained panel of consumers. The sensory assessment results are given below:

**Table 3**

| **Example** | **Immediately After - Flavour Intensity** | **Standard error** |
|---|---|---|
| **A** | 37.26 | 3.8 |
| **1** | 39.98 | 4.7 |
| **B** | 33.81 | 3.2 |

**Table 4**

| **Example** | **10 minute after - Flavour Intensity** | **Standard error** |
|---|---|---|
| **A** | 31.37 | 3.4 |
| **1** | 32.50 | 3.6 |
| **B** | 25.37 | 3.2 |

**Table 5**

| **Example** | **Overall Freshness Score** | **Standard error** |
|---|---|---|
| **A** | 3.3 | 0.3 |
| **1** | 3.7 | 0.5 |
| **B** | 3.3 | 0.3 |

**Table 6**

| **Example** | **Pleasantness of Taste** | **Standard error** |
|---|---|---|
| **A** | 3.2 | 0.3 |
| **1** | 3.6 | 0.4 |
| **B** | 3.5 | 0.3 |

The antimicrobial results for the formulations are shown below:

**Table 7**

| **Sample** | **Mean log₁₀ bacteria** | **Statistical Group** |
|---|---|---|
| Water | 6.4 | A |
| Zinc-free tablet | 5.3 | B |
| Example B | 4.5 | C |
| Example 1 | 4 | C, D |
| Example A | 3.6 | D |

Where it can be seen that the claimed example has antimicrobial efficacy equivalent to the single and double salt controls.

It is thus demonstrated that the examples according to the invention provide a high freshness score yet still have a good level of flavour intensity and a pleasant taste.

## Claims

1. A solid oral care composition comprising at least three zinc salts:
i) the first at 20°C having a solubility great than 500g/litre in water and is zinc sulphate 7H₂O.
ii) the second at 20°C having a solubility from 0.05g/litre to 500g/litre in water and is zinc citrate.
iii) the third at 20°C having a solubility less than 0.05g/litre in water and is zinc oxide.

2. An oral care composition according to claim 1 in which the total level of zinc salts/oxides is from 0.1 to 5 wt% of the total composition.

3. An oral care composition according to any preceding claim in which the composition is in the form of a tablet.

4. An oral care composition according to any preceding claim in which the weight ratio of the first zinc salt to the second zinc salt is from 1:2 to 1:6.

5. An oral care composition according to any preceding claim in which the weight ratio of the first zinc salt to the third zinc salt/oxide is from 1:6 to 1:15.

6. An oral care composition according to any preceding claim which further comprises calcium carbonate.

7. An oral care composition according to any preceding claim in which the calcium carbonate has a particle size (D50) ranging from 0.1 to 30 microns, preferably 0.1 to 15 microns, most preferably 2 to 10 microns as measured by Malvern mastersizer.

8. An oral care composition according to any preceding claim which further comprises a foaming agent selected from the group consisting of anhydrous surfactants selected from sodium lauryl sulfate, sodium lauryl sulfoacetate, cocamidopropyl betaine, sodium alpha olefin sulfonate, dioctyl sodium sulfosuccinate, sodium dodecyl benzene sulfonate and mixtures thereof.

9. A solid oral care composition according to any preceding claim, in which the composition also includes one or more organic polyols selected from glycerol, sorbitol, malitol, xylitol and mixtures thereof.

10. A oral care composition according to any preceding claim which is in the form of a compressed tablet.

11. An oral composition according to any of the preceding claims for use in a method for cleaning the teeth comprising the step of placing in the mouth the oral care composition, followed by chewing said oral care composition.

## Patentansprüche

1. Feste Mundpflegezusammensetzung, umfassend mindestens drei Zinksalze, wobei:
i) das erste bei 20 °C eine Löslichkeit von mehr als 500 g/Liter in Wasser aufweist und Zinksulfat 7H2O ist;
ii) das zweite bei 20 °C eine Löslichkeit von 0,05 g/Liter bis 500 g/Liter in Wasser aufweist und Zinkcitrat ist;
iii) das dritte bei 20 °C eine Löslichkeit von weniger als 0,05 g/Liter in Wasser aufweist und Zinkoxid ist.

2. Mundpflegezusammensetzung nach Anspruch 1, bei der der Gesamtgehalt an Zinksalzen/-oxiden 0,1 bis 5 Gew.-% der Gesamtzusammensetzung beträgt.

3. Mundpflegezusammensetzung nach irgendeinem vorhergehenden Anspruch, bei der die Zusammensetzung in Form einer Tablette vorliegt.

4. Mundpflegezusammensetzung nach irgendeinem vorhergehenden Anspruch, bei der das Gewichtsverhältnis des ersten Zinksalzes zum zweiten Zinksalz 1:2 bis 1:6 beträgt.

5. Mundpflegezusammensetzung nach irgendeinem vorhergehenden Anspruch, bei der das Gewichtsverhältnis des ersten Zinksalzes zum dritten Zinksalz/ -oxid 1:6 bis 1:15 beträgt.

6. Mundpflegezusammensetzung nach irgendeinem vorhergehenden Anspruch, die ferner Calciumcarbonat umfasst.

7. Mundpflegezusammensetzung nach irgendeinem vorhergehenden Anspruch, bei der das Calciumcarbonat eine Teilchengröße (D50) im Bereich von 0,1 bis 30 Mikron, bevorzugt 0,1 bis 15 Mikron, am meisten bevorzugt 2 bis 10 Mikron aufweist, gemessen durch Malvern-Masterizer.

8. Mundpflegezusammensetzung nach irgendeinem vorhergehenden Anspruch, die ferner ein Schäummittel umfasst, das ausgewählt ist aus der Gruppe bestehend aus wasserfreien Tensiden, ausgewählt aus Natriumlaurylsulfat, Natriumlaurylsulfoacetat, Cocamidopropylbetain, Natrium-alphaolefinsulfonat, Dioctylnatriumsulfosuccinat, Natriumdodecylbenzolsulfonat und Mischungen davon.

9. Feste Mundpflegezusammensetzung nach irgendeinem vorhergehenden Anspruch, wobei die Zusammensetzung auch ein oder mehrere organische Polyole, ausgewählt aus Glycerin, Sorbit, Malit, Xylit und Mischungen davon, umfasst.

10. Mundpflegezusammensetzung nach irgendeinem vorhergehenden Anspruch, die in Form einer Presstablette vorliegt.

11. Mundpflegezusammensetzung nach irgendeinem der vorhergehenden Ansprüche zur Verwendung in einem Verfahren zum Reinigen der Zähne, umfassend den Schritt des Platzierens der Mundpflegezusammensetzung in den Mund, gefolgt von Kauen der Mundpflegezusammensetzung.

## Revendications

1. Composition solide pour le soin oral comprenant au moins trois sels de zinc :
i) le premier à 20°C ayant une solubilité supérieure à 500 g/litre dans de l'eau et est le sulfate de zinc 7H₂O.
ii) le second à 20°C ayant une solubilité de 0,05 g/litre à 500 g/litre dans de l'eau et est le citrate de zinc.
iii) le troisième à 20°C ayant une solubilité inférieure à 0,05 g/litre dans de l'eau et est l'oxyde de zinc.

2. Composition pour le soin oral selon la revendication 1, dans laquelle la teneur totale en sels/oxydes de zinc est de 0,1 à 5 % en masse de la composition totale.

3. Composition pour le soin oral selon l'une quelconque des revendications précédentes, dans laquelle la composition est dans la forme d'un cachet.

4. Composition pour le soin oral selon l'une quelconque des revendications précédentes, dans laquelle le rapport massique du premier sel de zinc au second sel de zinc est de 1:2 à 1:6.

5. Composition pour le soin oral selon l'une quelconque des revendications précédentes, dans laquelle le rapport massique du premier sel de zinc au troisième sel/oxyde de zinc est de 1:6 à 1:15.

6. Composition pour le soin oral selon l'une quelconque des revendications précédentes qui comprend de plus du carbonate de calcium.

7. Composition pour le soin oral selon l'une quelconque des revendications précédentes, dans laquelle le carbonate de calcium présente une taille de particule (D50) de 0,1 à 30 microns, de préférence de 0,1 à 15 microns, encore mieux de 2 à 10 microns comme mesurée par un Mastersizer Malvern.

8. Composition pour le soin oral selon l'une quelconque des revendications précédentes qui comprend de plus un agent moussant choisi dans le groupe consistant en tensioactifs anhydres choisis parmi le laurylsulfate de sodium, laurylsulfoacétate de sodium, cocamidopropylbétaïne, oléfine-alpha sulfonate de sodium, sulfosuccinate de dioctylsodium, dodécylbenzènesulfonate de sodium et mélanges de ceux-ci.

9. Composition solide pour le soin oral selon l'une quelconque des revendications précédentes, dans laquelle la composition comprend également un ou plusieurs polyols organiques choisis parmi le glycérol, sorbitol, malitol, xylitol et mélanges de ceux-ci.

10. Composition pour le soin oral selon l'une quelconque des revendications précédentes qui est dans la forme d'un cachet comprimé.

11. Composition pour le soin oral selon l'une quelconque des revendications précédentes destinée à une utilisation dans un procédé pour nettoyer les dents comprenant l'étape consistant à placer dans la bouche la composition pour le soin oral, puis à mâcher ladite composition pour le soin oral.
